(19) Europäisches Patentamt
European Patent Office
Office européen des brevets

(11) Publication number: **0 398 925 B1**

(12) # EUROPEAN PATENT SPECIFICATION

(45) Date of publication of patent specification: **20.10.93**

(51) Int. Cl.[5]: **A61K 31/56**, A61K 31/715, C08B 37/16

(21) Application number: **89901794.1**

(22) Date of filing: **17.01.89**

(86) International application number: **PCT/US89/00175**

(87) International publication number: **WO 89/06536 (27.07.89 89/16)**

(54) **GROWTH INHIBITING AGENT AND THE USE THEREOF.**

(30) Priority: **19.01.88 US 145407**
**10.01.89 US 295638**

(43) Date of publication of application: **28.11.90 Bulletin 90/48**

(45) Publication of the grant of the patent: **20.10.93 Bulletin 93/42**

(84) Designated Contracting States: **AT BE CH DE FR GB IT LI LU NL SE**

(56) References cited:

| | |
|---|---|
| EP-A- 0 188 821 | EP-A- 0 197 571 |
| WO-A-50/2767 | FR-A- 2 484 252 |
| JP-A-53 109 953 | JP-A-60 114 75 |
| US-A- 4 020 160 | US-A- 4 066 829 |
| US-A- 4 247 535 | US-A- 4 258 180 |
| US-A- 4 383 992 | US-A- 4 596 795 |
| US-A- 4 727 064 | US-A- 4 757 056 |
| US-A- 4 783 446 | |

**J. Cell. Biol., vol. 107, no. 6, part 3, p. 579a, abstract no. 3277; J. Folkman et al.**

(73) Proprietor: **CHILDREN'S HOSPITAL CORPORATION**
**300 Longwood Avenue**
**Boston, MA 02115(US)**

Proprietor: **THE TRUSTEES OF THE UNIVERSITY OF PENNSYLVANIA**
**133 South 36th Street**
**Philadelphia, Pennsylvania 19104(US)**

(72) Inventor: **Folkman, Moses Judah**
**18 Chatham Circle**
**Brookline, MA 02146(US)**
Inventor: **Weisz, Paul Burg**
**3 Delaware Rim Drive**
**Yardley, PA 19067(US)**

(74) Representative: **Warcoin, Jacques et al**
**Cabinet Régimbeau**
**26, avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 398 925 B1

J. of Pharmac. Sciences, vol. 72, no. 10, Oct. 1983, pp. 1215-1217, American Pharmac. Ass.; S.G. Frank et al.

J. of Pharmac. Sciences, vol. 74, no. 9, September 1985, pp. 987-990, American Pharmac. Ass.; J. Pitha et al.

Dialog Information Services, File 155 (Medline), accession no. 06630385; D. Ingber et al.

J. Natl. Cancer Inst., vol. 75, no. 2, August 1985, pp. 353-359; W.D. Klohs et al.

Science, vol. 230, no. 4732, 20th Dec. 1985, pp. 1375-1378; R. Crum et al.

Science, vol. 243, no. 4897, 17th March 1989, pp. 1490-1493; J. Folkman et al.

Dialog Information Services, File 155 (Medline), accession no. 04371364; J. Gross et al.

Chemical Abstracts, vol. 83, no. 9, 1 Sepr. 1975, abstract no. 83:79544a

## Description

This invention is concerned broadly with the inhibition of pathological or undesired cell or tissue growth in mammals by use of a new growth-inhibiting composition. More specifically, the present invention is directed to a growth-inhibiting composition comprising a highly soluble cyclodextrin derivative and a latent or active growth-inhibiting compound, and to the use of this composition to inhibit undesired or pathological growth, including angiogenesis which is associated with, inter alia, the growth of malignant tumors.

Angiogenesis, the induction of growth of new capillary blood vessels, is important in normal processes such as development of the embryo, formation of the corpus luteum and healing of wounds. It is also an important component in pathological processes such as chronic inflammation, certain immune responses, and neoplasia. It is now accepted that angiogenesis is induced by most malignant tumors and that it is necessary for their continued growth and survival. It is also recognized that angiogenesis is a major component of a number of ophthamological pathologies including such as diabetic retinopathy, retrolental fibroplasia and neovascular glaucoma. Additionally, angiogenesis is now recognized as a major component in other non-neoplastic pathological conditions including rheumatoid arthritis, in which abnormal capillary growth can destroy joint cartilage; hemanogiomas, in which abnormal capillary proliferation appears in newborns and can persist for up to 2 years; angiofibromas which develop in the nasopharynx; and psoriasis, in which excessive proliferation and shedding may be dependent on abnormal capillary growth in the dermis [see Folkman and Klagsbrun, Science 235:442 (1987)].

It has been previously discovered that heparin (or heparin fragments) and cortisone will co-act together to inhibit angiogenesis. When administered together to mice with certain kinds of tumors, this combination can inhibit the generation of essential capillary vessels that support tumor growth, and can cause the collapse of the blood supply which supports the tumors. A review of the history of this discovery and of related subject matter is contained in the publication "How is Blood Vessel Growth Regulated in Normal and Neoplastic Tissue?" (G.H.A. Clowes Memorial Award Lecture), Judah Folkman, Cancer Research, 46:467 (1986).

Cortisone is an anti-inflammatory agent that by itself does not have the ability to inhibit capillary growth. It has been reported in Shubik et al., J. Nat'l Cancer Inst. 57:769 (1976) that 6 $\alpha$-methyl prednisolone partially suppressed tumor angiogenesis in hamster cheek pouches under certain conditions, but tumor growth was not stopped. Many other publications have reported continued growth of tumors even in the presence of large amounts of cortisone. It has also been reported [Gross et al., Proc. Nat'l. Acad. Sci. USA 78:176 (1981)] that medroxyprogestrone, dexamethasone and to a lesser extent cortisone, inhibited tumor angiogenesis in rabbit corneas, while estradiol and testosterone were ineffective.

Aside from cortisone, certain other steroids are now known to successfully suppress angiogenesis when administered together with heparin or certain heparin fragments. The effective steroids have been referred to as "heparin-dependent" because heparin was (until now) unique in its effect. The findings and the character of desirable angiostatic steroids has been discussed in "A New Class of Steroids Inhibits Angiogenesis in the Presence of Heparin or a Heparin Fragment", R. Crum, S. Szabo and J. Folkman, Science 230:1375 (1985); and in "Angiostatic Steroids", J. Folkman and D.E. Ingber, Annals of Surgery, 206:374 (1987).

Heparin, a mucopolysaccharide, is a constitutent of various tissues, especially liver and lung, and mast cells in several mammalian species. Chemically, it has been described as an $\alpha$, $\beta$ glycosidically linked sulfated copolymer of D-glucosamine and D-glucuronic acid. However, although heparin has been used clinically as an anticoagulant for half a century, both the exact structure of heparin and the precise nature by which it acts in blood anti-coagulation have not been discovered. Much of the difficulty in determining the structure of heparin results from its complexity and the fact that it is not a homogeneous, well-defined substance. Heparin is polydisperse with a molecular weight range from about 5,000 to 40,000. Within a given chain, there are also structural variations such as the varying degrees of sulfation, N-acetylation and C-5 epimerization in the uronic acid residue.

A major disadvantage in the use of heparin with a steroid to inhibit angiogenesis results from the fact that heparins manufactured by different processes and different companies revealed quite different antiangiogenic activities despite similar anticoagulant activities. The precise composition of commercial heparin apparently varies depending on its source and method of manufacture. While some heparins may be combined with cortisone to inhibit angiogenesis, other heparins are not effective as such. In fact, some heparins in order to be effective may be required in such high doses that administration may cause problems due to the anticoagulant activity of heparin. A second disadvantage is that while heparins apparently can inhibit the growth of responsive tumors when administered in the proper dose range and proper ratio to steroid, and even , promote regression at somewhat higher doses and ratios; heparins can

also cause resumption of rapid tumor growth when administered at even higher dose levels and ratios to steroid. The apparent presence of both positive and negative regulators of angiogenesis in heparin may create problems in properly administering the drug. An additional disadvantage derives from the anticoagulant activity of heparin, restricting its use to low dosage levels or to oral administration in order to avoid bleeding. Finally because heparin cannot penetrate the corneal membrane, it cannot be topically applied to the exterior of the cornea for its desired antiangiogenic activity.

Cyclodextrins (hereinafter referred to for convenience as CD or CDs for the singular and the plural, respectively) are cyclic oligosaccharides consisting of at least six glucopyranose units. Although CDs with up to twelve glucopyranose units are known, only the first three homologs have been studied extensively. These compounds have the simple, well-defined chemical structure shown in FIG. 1(A). The common designations of the lower molecular weight α-, β-and γ-CDs are used throughout this specification and will refer to the chemical structure shown in FIG. 1(A) wherein $n = 6$, 7, or 8 glucopyranose units, respectively. The initial discovery of the CDs as degradation products of starch was made at about the turn of the century, and Schardinger showed that these compounds could be prepared by the action of Bacillus macerans amylase upon starch. In older literature, the compounds are often referred to as Schardinger dextrins. They are also sometimes called cycloamyloses.

Topographically, the CDs may be represented as a torus, as shown in FIG. 1(B), the upper rim of which is liked with primary -$CH_2OH$ groups, and the lower rim with secondary hydroxyl groups. Coaxially aligned with the torus is a channel-like cavity of 5, 6 or 7.5 A.U. diameter for the α-, β-, and γ-CDs, respectively. These cavities make me cyclodextrins capable of forming inclusion compounds with hydrophobic guest molecules of suitable diameters.

A reasonably large number of CD derivatives have been prepared and described in the literature. In general, these chemically modified CDs are formed by reaction of the primary or secondary hydroxyl groups attached to carbons 2, 3 or 6 [FIG. 1(A)], without disturbing the α (1 --> 4) hemiacetal linkages. A review of such preparations is given in "Tetrahedron Report Number 147, Synthesis of Chemically Modified Cyclodextrins", A.P. Croft and R.A. Bartsch, Tetrahedron 39(9):1417-1474 (1983) (hereinafter referred to as "Tetrahedron Report No. 147").

In particular, a-, β-, and γ-CD sulfates (Na salt) are shown as Compound Nos. 207, 208, and 209 in Tetrahedron Report No. 147, (supra) Table 26, p.1456. U.S. Patent 2,923,704 to Berger describes the preparation of cycloamylose sulfates. U.S. Patent 4,020,160 to Berstein et al. and U.S. Patent Nos. 4,247,535 and 4,258,180 to Lewis et al. disclose the use of modified cyclodextran sulfates as complement inhibitors. U.S. patent 4,383,992 to Lipari describes the preparation of a water-soluble inclusion compound of a steroid and unmodified β-cyclodextrin. U.S. patent 4,596,795 to Pitha discloses the administration (by the sublingual or buccal route) of sex hormones, particularly testosterone, progesterone and estradiol in the form of their inclusion compounds with hydroxypropyl-β-CD or poly-β-CD. None of the foregoing references are believed to show or make obvious applicants' invention as described and claimed herein.

We have now found that certain simple and very water-soluble derivatives of the cyclodextrins when administered together with a latent growth-inhibiting steroid such as cortisone or hydrocortisone or with a non-steroidal growth-inhibiting organic compound effectively inhibit angiogenesis without exhibiting the undesirable properties of heparin.

One of the objects of the present invention is to provide a novel composition including a derivative of a cyclodextrin and a growth-inhibiting compound, which composition is effective for inhibiting cell or tissue growth, especially angiogenesis, or for treating tumors, in mammals, including humans.

Another object of the invention is to provide pharmaceutical preparations containing a highly water soluble cyclodextrin derivative, especially a cyclodextrin sulfate salt, and one or more steroid compounds.

These and other objects, aspects and advantages of the present invention will become apparent to those skilled in the art upon reviewing the following description and appended claims.

This invention provides a composition for inhibiting undesired or pathological cell or tissue growth (including angiogenesis) in mammals, including humans, said composition comprising active agents consisting of (1) a very water-soluble derivative of α-,β- γ-cyclodextrin in combination with (2) a latent growth-inhibiting steroid or a non-steroidal growth-inhibiting organic compound having inherent antiangiogenic activity.

This invention further provides the use of a composition consisting of (1) a derivative or α-,β or γ-cylodextrin in combination with (2) a latent growth-inhibiting steroid or a non-steroidal growth-inhibiting organic compound having inherent antiangiogenic activity fo the preparation of a medicament for inhibiting undesired or pathological cell or tissue growth, including angiogenesis, in mammals, including humans.

This invention further provides the use of a composition consisting of (1) a derivative of α-,β- or γ-cyclodextrin in combination with (2) a latent growth-inhibiting steroid or non-steroidal growth-inhibiting

organic compound having inherent antiangiogenic activity, for the preparation of a medicament for inhibiting angiogenesis in mamals, including humans.

This invention further provides a product containing (1) a derivative of α, β or γ-cyclodextrin and (2) a latent growth-inhibiting organic compound having inherent antiangiogenic activity as a combined preparation for simultaneous, separate or sequential use for inhibiting the pathological growth of smooth muscle cells in mammals, including humans.

The present invention may be more fully understood by reference to the following detailed description of the invention, examples of specific embodiments of the invention and appended figures in which:

FIG. 1(A and B) is a schematic representation of: (A) the chemical structure of α-, β and γ- cyclodextrins; and (B) of the three-dimensional shape of these cyclodextrins.

FIG. 2 (A and B) graphically illustrates the effect of β-cyclodextrin tetradeca sulfate (β-CD-TDS) or heparin on growth of (A) rat aortic smooth muscle cells and (B) calf aortic smooth muscle cells in tissue culture.

FIG. 3(A - D) is a photographic representation of the effect of topically administered agents on endotoxin-induced capillary vessel development in the rabbit cornea. Effects of (A) Endotoxin alone (i.e., Control Group); (B) hydrocortisone alone, (Group 2); (c) β-CD-TDS + hydrocortisone, (Group 3); and (D) β-CD-TDS alone (Group 4) are shown. See text for experimental details.

FIG. 4 graphically illustrates the effect of implantation of sustained release polymers incorporating various agents on endotoxin-induced capillary blood vessel elongation in the rabbit cornea. [O] = Endotoxin alone, (Control Group); [●] = β-CD-TDS + Cortexolone, (Group 2); [△] = Cortexolone alone, (Group 3); and [◻] = β-CD-TDS alone, (Group 4). See text for experimental details.

The present inventors searched for a compound other than heparin, which would not have the disadvantages of heparin, but which when combined with a steroid would be effective for suppressing undesired or pathological cell or tissue growth including angiogenesis and, therefore, would be effective, inter alia, for controlling or eliminating tumors in mammals, including humans.

Because of the known ability of cyclodextrins including β-CD to form water soluble inclusion compounds with steroids, we attempted to make use of mixtures of unmodified CDs with hydrocortisone. However, it was discovered that such mixtures had no effect for suppressing angiogenesis, as shown by Examples 18-20 (infra, Section 7).

Quite surprisingly, we discovered that highly water-soluble salts of cyclodextrin in combination with a steroid, such as hydrocortisone, were effective for inhibiting angiogenesis. In particular, β-CD tetradeca sulfate (β-CD-TDS) was found to be very effective. In other words, a composition comprising a steroid and a water-soluble cyclodextrin derivative is effective for inhibiting undesired cell or tissue growth, including angiogenesis, and for treating tumors in mammals.

The CD sulfates and other highly water-soluble derivatives discussed herein have been found to be reproducible in their effect in the chick chorioallantoic membrane (CAM) assay described below. This assay has been employed as a model assay to detect angiogenic activity of various substances. [Klagsbrun et al., Cancer Res. 36:10(1976)]. Three separate batches of β-CD-TDS prepared by the method described in Example 1(A) were compared in the CAM assay. Results for the three separate batches were indistinguishable. The highly water-soluble CD derivatives mimic the advantageous features of antiangiogenic heparin without its disadvantages. These findings also set aside the notion that heparin is unique in its role in suppressing angiogenesis when combined with a suitable steroid.

We have also found that use of a highly water-soluble CD derivative such as β-CD-TDS together with a non-steroidal compound that inhibits growth in the absence of exogenous heparin, i.e., a compound that inherently has some antiangiogenic activity, surprisingly potentiates the antiangiogenic activity of such compound. Examples of such non-steroidal compounds include, but are not limited to proline analogs such as L-2-azetidinecarboxylic acid,

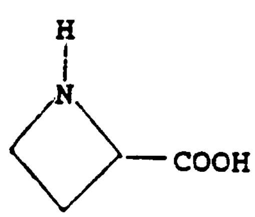

(see, infra, Example 24), cishydroxyproline, or 3,4-dehydroproline, and transretinoic acid. L-2-azetidinecarboxylic acid is described in The Merek Index, 10th edition (1983) under Compound 911. [See Ingber and Folkman, Lab. Investig. 59:44 (1988) for a description of such non-steroidal growth inhibiting compounds which are proline analogs.]

To clearly distinguish the steroids (which in the absence of exogenous heparin, have no inherent antiangiogenic activity) from such non-steroidal growth-inhibitory compounds, the qualifying phrase "latent growth-inhibiting" is used herein. The adjective "non-steroidal" as used herein means a compound in which carbon ring structure characteristic of a sterol is absent.

Highly water-soluble CD derivatives bearing non-ionic and/or ionic substituents are useful for inhibiting undesired growth according to the present invention. Suitable highly water-soluble CD derivatives include α, β and γ CD derivatives having non-ionic substituents including but not limited to alkyl substituents such as methyl and ethyl as well as those in which a number of hydroxyl groups are replaced by other groups so as to increase the hydrophilic activity of the CD. Such groups may include, esters, ethers, thioesters, thioethers, carboxylic acids or other groups which convey hydrophilic activity by way of polar or hydrogen bonding constituents or they may include partial hydroxyl substitution that allows better hydrogen bonding involving the remaining hydroxyl groups.

The CD derivatives useful in the present invention are highly hydrophilic and therefore very water soluble. Without wishing to be bound by theory, we believe that a highly hydrophilic character is important to allow interaction with cellular surfaces. We also believe a very high water solubility of the derivative is an important factor which cooperatively interacts with the inherent complexing ability of the CD structure to provide effective inhibition of angiogenesis with an exogenous steroid, as provided by this invention. We believe that the hydrophlilic activity is roughly indicated by the affinity to water, as measured by water solubility. It is important to measure the same at 0 °C since at higher temperatures the most suitable derivative have solubilities so high that meaningful measurements are difficult.

As shown in Table III (Examples 18-22, infra, Section 7), the most soluble derivatives (measured at O °C) show the highest antiangiogenic activity. Of the CDs, the β-CD derivatives appear to be most effective. In general, useful potency is evident at a solubility, measured at O °C, of at least about 15 g/100 ml in distilled water, preferably at least about 20 g/100 ml, more preferably about 30 g/100 ml. All solubility measurements referred to herein relate to the solubility of the substantially anhydrous derivatives, and when these are salts, to the anhydrous sodium form. The term "very soluble" as used herein refers to a solubility of at least 15 g/100 ml measured as described above.

It is contemplated that very water-soluble CD derivatives bearing tonic and/or non-ionic substituents may in some instances have advantageous properties. Although highly water-soluble derivatives in general are believed useful, salt derivatives are preferred.

The phrase "salt derivative" as used herein means an ionic compound derived from a CD by reaction with a suitable reagent. The preferred salt derivatives are provided by a cyclodextrin having substituents selected from the group consisting of sulfate, phosphate, carboxylate and mixtures thereof associated with a non-toxic, physiologically acceptable cation. Many of said preferred derivatives are known compounds. (See, Tetrahedron Report Number 147, supra). But many potentially useful forms may be variants, structurally or chemically of known compounds. They also may possess several different substituents such as is the cake of the cyclodextrin propoxyl sulfate of Example 1D, which we believe has not previously been reported. Some of the preferred salt forms of the derivatives are the sodium and potassium forms, since these tend to impart increased water solubility to organic anions. The salt derivatives useful herein will exibit electrolytic conductivity and osmotic properties characteristic of electrolytes and polyelectrolytes when in aqueous solution. A particularly preferred salt derivative is β-cyclodextrin tetradeca sulfate (β-CD-TDS).

The α-, β- and γ-CD sulfate salts are all usable in the presently claimed invention. β-CD sulfate salts are preferred. Various degrees of sulfation per glucose unit can be employed, such as average of one sulfate group per two glucose units or two sulfate groups per glucose unit. Cyclodextrins having about two sulfate groups per glucose unit are preferred. Especially preferred is β-CD-TDS which has an average of two sulfate groupes per glucose unit.

As a preferred embodiment, the steroid has 17-alpha and 21-hydroxyl group, 3- and 20- one groups and in the 16-position hydrogen, hydroxy or a methyl group and non-toxic, physiologically acceptable carboxylates, acetal, ketals and phosphates thereof.

Among the steroids which are effective and can be utilized in the presently claimed invention are the following:

17 alpha, 21-dihydroxy-4-pregene-3,11,20-trione and its 21-acetate (or cortisone);
11 alpha, 17,21-trihydroxypregn-4-ene-3,20-dione (or 11 alpha hydrocortisone);
11 beta, 17 alpha, 21-trihydroxypregn-4-ene-3,20-dione (or hydrocortosone);

17 alpha, 21-dihydroxypregna-4,9(11)-diene-3,20-dione;
15 alpha, 17 alpha, 21-trihydroxy-4-pregnene-3,20-dione;
16 alpha, 17 alpha, 21-trihydroxy-6 alpha-methylpregn-4-ene-3,20-dione-21-acetate-16,17 cyclic ketal of acetone;
6 alpha-fluoro-17 alpha, 21-dihydroxy-16 beta-methyl-pregna-4,9(11)-dinene-3,20-dione;
6 alpha-fluoro-18 alpha,21-dihydroxy-16 beta-methyl-pregna-4,9(11)-diene-3,20-dione-17,21-diacetate;
6 beta, 17 alpha, 21-trihydroxypregn-4-ene-3,20-dione;
17 alpha, 21-dihydroxypregn-4-ene-3,20-dione-21-acetate;
17 alpha, 21-dihydroxypregn-4-ene-3,20-dione (or Cortexolone);
9 beta, 11 beta-epoxy-17 alpha, 21-dihydroxy-2 alphamethylpregn-4-ene-3,20-dione-21-acetate;
17 alpha, 21-dihydroxy-16 alpha-methylpregn-4-ene-3,20-dione;
9 alpha, 11 beta-dichloro-17 alpha, 21-dihydroxypregn-4-ene-3,20-dione-21-acetate
17 alpha, 21-dihydroxy-6 alpha, 16 alpha-dimethylpregn-4-ene-3,20-dione-21-acetate;
17 alpha, 21-dihydroxy-16 alpha-methylpregna-4,9(11)-diene-3,20-dione-21-acetate;
17 alpha, 21-dihydroxy-16 beta-methylpregna-4,9(11)-diene-3,20-dione-21-benzoate;
6 alpha-fluoro-17 alpha 21-dihydroxy-16 beta-methylpregna-4,9(11)-diene-3,20-dione-17-acetate-21-benzoate;
17 alpha, 21-dihydroxy-15 beta-methylpregna-1,4,9(11)-triene-3,20-dione-17-succinate sodium monohydrate;
9 alpha-fluoro-11 beta, 16 alpha, 17 alpha, 21-tetrahydroxy-pregn-4-ene-3,20-dione-16,21-diacetate;
17 alpha, 21-dihydroxy-16 alpha-methylpregna-1,4,9(11)-triene-3,20-dione-21-succinate sodium monohydrate;
6 alpha-fluoro-17 alpha, 21-dihydroxy-16 beta-methyl-pregna-1,4,9(11)-triane-3,20-dione-21-succinate sodium;
desoxycorticosterone;
testosterone;
estrone.

More preferred are those steroids which lack glucocorticoid end mineralo-corticoid activity, since such activity is an undesired effect and limits the dose size or extent of use of the steroid for the purpose of the present invention. Among such more preferred steroids are 11 alpha, 17-21-trihydroxypregn-4-ene-3,20-dione (or alphahydrocortisone), 17 alpha, 21-dihydroxypregn-4-ene-3,20-dione (11-desoxycortisol or Cortexolone), and 17 alpha, 21-dihydroxypregna-4,9(11)-diene-3,20-dione.

None of the steroids themselves effectively inhibits angiogenesis nor causes regression of tumors in the absence of a water-soluble cyclodextrin derivative of the present invention.

As taught by the present invention, the growth-inhibitory activity of non-steroidal organic compounds is potentiated by combination with a water-soluble cyclodextrin derivative. Among the non-steroidal growth-inhibiting organic compounds which are effective and can be utilized in the presently claimed invention are the following: proline analogs such as L-2 azetidinecarboxylic, cishydroxyproline, and 3,4-dehydroproline and transretinoic acid acid.

Additionally, any non-steroidal organic compound which in combination with a cyclodextrin derivative demonstrates growth inhibiting activity in either of the bioassays described below can be utilized according the presently claimed invention.

Several bioassays have been developed to estimate the angiogenic-inhibiting potency, if any, of a substance. The rabbit cornea is the basis of one of these methods. The cornea is avascular. A small pocket can be made in it and a tumor implant can be inserted while the rabbit is anesthetized. The tumor is separated from the vascular bed of the host. New capillary blood vessels will grow in a linear manner toward the tumor, and the rate of vessel growth can be measured. [For a more detailed description of this assay, see, Gimbrone et al., J. Nat'l Cancer Inst. 52:413 (1973).

A more economic bioassay makes use of the chorioallantoic membrane of the chick embryo. This test will for convenience be referred to hereinafter as the "CAM assay". For a more detailed description of the CAM assay, see Folkman et al., Science 221:719 (1983). A typical CAM assay, such as used for the evaluations in the examples in Section 7, *infra*, employs 16 eggs per experiment. A 2 mm diameter disk of methylcellulose containing the test substance is applied to the chorioallantoic membrane of a 6-day chick embryo, cultured in a Petri dish, in a humidified incubator with 3% carbon dioxide. Two days later (8-day embryo), the membrane is examined under a stereomicroscope at six- to ten-fold magnification. Inhibition of angiogenesis by the test substance is evidenced by the development of an avascular zone around the methylcellulose disc. An avascular zone of 4 mm is graded as (+ +) and an avascular zone of 2 mm is graded as (+). The potency of the inhibition at the 2 mm and 4 mm zone(s) are expressed as the percentage of the total number of eggs (usually 16) in the test that were rated (+ +) or (+), i.e., the % of

"successes". A rating of zero % reflects absence of inhibition or the test substance under the test conditions.

The sustained release methylcellulose discs are prepared by dispersing appropriate amount(s) of the test substance of substances in an 0.45% aqueous solution of methylcellulose, and depositing 10 microliter aliquots of the resulting solution in a Teflon mold, followed by air drying for about one hour in a laminar flow hood.

A very advantageous feature of the CAM assay is the very high sensitivity of the chick embryo to toxic substances. Moreover, the lack of toxicity of a substance in the CAM assay has been correlated with lack of toxicity of such substance when administered to other animals.

The composition of the present invention is useful for inhibiting undesired cell and tissue growth, including angiogenesis. Of course, the composition of the present invention comprising a water soluble derivative of an $\alpha$-, $\beta$-or $\gamma$-CD and a steroid is to be administered to mammals including humans in need of such treatment. For example, mammals with tumors are in need of treatment with the composition of the present invention. While not completely understood, it is believed that treatment with the composition of the present invention inhibits the creation of new capillaries necessary for tumor growth. This results in the tumor having an insufficient supply of nutrients essential for its growth or even for its vitality. Thus, tumors in mammals including humans, when treated in accordance with the present invention, do not grow and may even lose their vitality and die. Among the tumors contemplated as responsive to the composition and methods of this invention are Reticulum Cell Sarcoma, Lewis Lung Carcinoma, B-16 Melanoma, and Bladder Carcinoma, etc.

Neither mature non-growing blood vessels nor vascular tissue appear to be affected by the treatment with the composition of the present invention. Inhibition of angiogenesis with the compositions of the present invention, in addition to its effect upon tumor regression and metastasis in tumor-bearing animals, may be effective in treating a number of other ailments.

The compositions of the present invention may further be used in a method for treatment of a number of other non-tumorous disorders which are characterized by pathological cell or tissue growth, including angiogenesis. Thus the invention provides compositions for treatment of mammals, including humans, afflicted with a number of non-neoplastic pathological conditions including rheumatoid arthritis, in which abnormal capillary growth can destroy joint cartilage; hemanogiomas, in which abnormal capillary proliferation appears in newborns and can persist for up to 2 years; angiofibromas which develop in the nasopharynx; psoriasis, in which excessive proliferation and shedding may be dependent on abnormal capillary growth in the dermis. Additionally, the present invention provides a method for treatment of a number of ophthalmological pathologies which are associated with undesired angiogenesis, including diabetic retinopathy, retrolental fibroplasia and neovascular glaucoma.

The present invention further provides compositions for inhibiting undesired smooth muscle cell development often observed following angioplasty or treatment to remove atherosclerotic plaques which occlude blood vessels.

According to one embodiment of the method of the present invention, the active agents are to be mixed together prior to administration so that the steroid compound or non-steroidal growth-inhibiting compound is administered in combination with the water-soluble cyclodextrin derivative. After the mixture is prepared, it may be administered orally or parenterally including, inter alia, topical application, intravenous, intra-arterial or subcutaneous injection, and including absorption as well as injection and introduction into body apertures or orifices.

Cortisone and its physiologically accepted non-toxic derivatives, such as the acetates, as well as many other steroids useful in the present invention, are only slightly soluble in water. However, when combined with the water-soluble cyclodextrin derivatives of the invention, the resulting complexes have increased water solubility. Accordingly, the composition of the present invention can easily be administered. The term "cortisone" and "hydrocortisone" and 11-$\alpha$ isomer of hydrocortisone as used in the present specification and claims are intended to include both the steroids themselves and their derivatives.

According to an alternate embodiment of the method of the invention, the active agents are each to be administered separately and the combination of the two agents forms in vivo. In this embodiment, the two active agents can be introduced separately either via the same or different routes of administration, so long as both agents are thus present simultaneously in vivo, permitting a complex mixture of the two active agents to form.

Dosages employed are limited only by the well-known limits of the administration of drugs individually for their usual effects, in the case of cortisone, hydrocortisone, or 11-$\alpha$ isomer. Since the CD derivatives useful herein have no anticoagulant effect and show no toxicity in the CAM test at dosages employed according to the method of the invention (see below), they may be administered percutaneously in dosages

at least as large as those acceptable for heparin. Oral dosage may be considerably higher.

The dose amount required to bring about arrest of tumor growth or regression of tumors varies depending upon the identity of the tumor, as does the length of time required to bring about arrest or regression of tumors. Tumor size at the beginning of treatment also affects the length of time for complete regression. Because administration of cortisone, with or without β-CD-TDS(Na), for example, may result in pulmonary infection after a number of days, it may be desirable to administer a suitable antibiotic as prophylaxis during treatment in accordance with the present invention. Such antibiotics can be mixed with the water-soluble cyclodextrin derivative and the steroid or non-steroidal growth-inhibiting agents of the invention and administered as a mixture or, alternatively, the antibiotics can be administered alone contemporaneously with the water-soluble cyclodextrins and growth-inhibiting agents of the invention either by the same or a different route of administration.

As shown in Table I, infra Section 7, it appears that a weight ratio greater than about 2:1 or a molar ratio of greater than about 0.4 of water-soluble CD derivative to steroid leads to a decrease of antiangiogenic activity. The preferred molar range and the molar ratio of CD derivative to angiostatic agent (steroidal or non-steroidal) is 0.004 to about 0.7. A more preferred useful range is 0.04 to 0.7. The latter range is particularly useful in topical applications, including as eye drops for ophthalmological uses. The amount of angiostat will generally be of the order of 0.1 to 10 mg/ml when admixed with the CD derivative, however, the absolute amount may depend on the mode and frequency of administration.

The effective compositions of this invention are best administered in a suitable carrier, which must be non-toxic and physiologically acceptable, such as water or normal saline solution. Compositions containing mixtures of the active agents or each of the active agents alone, either dry or in a suitable carrier, can be employed.

## 7. EXAMPLES

The following examples are provided to illustrate this invention. All amounts and proportions shown are by weight unless explicitley stated to be otherwise. For the CAM assay, however, % refers to the number of "successes". (See Section 6.3, above).

### Examples 1 (A-D)

This example illustrates the best mode known to us for preparing and purifying cyclodextrin sulfates.

#### (A) β CD-TDS(Na):

β-cyclodextrin (99% pure dihydrate) was purchased from Chemalog (a division of General Dynamics Corp.), South Plainfield, New Jersey.

5.0 grams of β-cyclodextrin (4.4 mmoles, i.e., about 92 meq -OH) was dissolved in 250 ml of dimethylformamide (DMF). To this solution was added 15.0 grams of $(CH_3)_3N\text{-}SO_3$ (108 mmoles) in a single portion and the reaction mixture was heated to 70 °C. After two hours at 70 °C, a gummy material began to precipitate. The reaction mixture was maintained at 70 °C with vigorous stirring, and then cooled to room temperature. The DMF layer was then decanted and discarded, and the solid residue was dissolved in 250 ml of water followed by addition of 75 ml of 30% sodium acetate. The mixture was stirred vigorously for 4 hours and then poured into 4000 ml of ethanol. After standing overnight, the fixture was filtered to recover the crystallized solids. The filter cake was washed with ethanol (absolute) followed by diethyl ether. The product was then dried under vacuum over $P_2O_5$. 10.3 grams of white powder was recovered. The product was hygroscopic.

The product was analyzed under conditions such that sorption of water was minimized. Elemental analysis gave the following results: C = 18.84, H = 2.65, S = 17.33 (Calculated for $C_6H_8O_{11}S_2Na_2$; C = 19.67, H = 2.19, S = 17.49). $[\alpha]^{22}_{D}$ = 75° (C = 2.63 in 0.5 M NaCl). The analysis corresponds to that expected for an average substitution of two hydroxyl groups for each glucopyranose unit, i.e., 14 hydroxyls per CD molecules. The calculated yield for such β-CD-TDS salt is 10.96 grams, about 6% higher than the observed 10.3 grams.

#### (B) α- and γ-CD-S (Na salt):

The procedure described above was used for these preparations except that 86 mmoles of $CH_3N\text{-}SO_3$ was used with α-CD, and 117 mmoles with the γ-CD.

The sulfated a-CD salt analyzed C = 18.76; H = 2.60; S = 16.22. This corresponds on average to a substitution of about 11.7 hydroxyl units per α-CD molecule.

The sulfated γ-CD salt analyzed C = 18.92; H = 2.69; S = 14.84. This corresponds on average to a substitution of about 14 hydroxyl groups per γ-CD molecule.

(C) β CD-$SO_4$ (Na salt) (7.1 wt% S):

1.0 g of β-cyclodextrin was dissolved into 50 ml of DMF. To this solution was added 883 mg of $(CH_3)_3N{\cdot}SO_3$ (7.2 equivalents). The solution was held at 75 °C for 12 hours, at which time no precipitate had formed. The reaction mixture was cooled to room temperature. To the solution was added 200 ml of ethanol. The resulting colloidal solution was then poured into 600 ml of diethyl ether. A white solid farmed in 2 hours. The solid was collected by filtration and then was dissolved in 30 ml $H_2O$. This solution was stirred for 2 hours. After stirring, the solution was poured into 900 ml of a 2:1 EtOH-$Et_2O$ solutlon. Crystals formed-over an 8 hour period. The crystals were collected and washed with $Et_2O$. The product was dried over $P_2O_5$ under vacuum. 1.18 g of powder was recovered. (72.4% yield).

Elemental analysis of the product showed C = 32.49; H = 4.99; and S = 7.06. This corresponds on average to a substitution of about 3.5 hydroxyls per β-CD molecule.

(D) β-CD-Propoxylate ~14 $SO_4$

β-CD-(hydroxy-n-propyl ether) was obtained from American Maize-Products Co. (Hammond, IN) and the procedure described above was used to prepare the sulfate salt, β-CD-(~4Pr~14 $SO_4$).

Examples 2-15

In these examples the angiogenesis-inhibiting potency of hydrocortisone with various dosage levels of β-CD-TDS prepared as in Example 1 was evaluated by the CAM assay. The methylcellulose discs all contained 60 µg of the steroid but the β-CD-TDS was varied from 100 µg down to 0.05 µg. The results are summarized in Table I. As can be seen from the data, angiogenesis inhibition persists with extremely low levels (0.05 µg) of the CD compound with no evidence of activation of angiogenesis at two thousand-fold higher concentration.

TABLE I

| Example No. | Hydrocortisone µg | Beta-CD-TDS µg | CAM Assay | |
|---|---|---|---|---|
| | | | (+ +) % | (+) % |
| 2 | 60 | 100 | - | 57 |
| 3 | " | 50 | 60 | 100 |
| 4 | " | 50 | 22 | 55 |
| 5 | " | 25 | 10 | 60 |
| 6 | " | 25 | 18 | 55 |
| 7 | " | 10 | 40 | 70 |
| 8 | " | 10 | 6 | 40 |
| 9 | " | 5 | 0 | 50 |
| 10 | " | 1 | 0 | 50 |
| 11 | " | 1 | 0 | 42 |
| 12 | " | 0.5 | 0 | 40 |
| 13 | " | 0.1 | 0 | 45 |
| 14 | " | 0.1 | 0 | 37 |
| 15 | " | 0.05 | 0 | 20 |

In contrast with these results, CAM tests made with 100 µg of α-, β- or γ- cyclodextrin with 50 µg of hydrocortisone all showed total absence of angiogenesis-inhibition [no successes at either the 1 mm zone (+) or the 2 mm zone (+ +) level].

Examples 16-17

Examples 16 and 17 illustrate the low, but useful, activity afforded by the sulfated α-CD and γ-CD as shown in Table II. Date for Examples 5 and 6 are included for comparison. All tests were made with 25 µg of the indicated CD sulfate and 50 µg of cortisone.

TABLE II

| Example No. | CD | Sulfur, Wt% | CAM Assay | |
|---|---|---|---|---|
| | | | (+) % | (++) % |
| 16 | Alpha | 16.2 | 8 | 0 |
| 17 | Gamma | 18.9 | 15 | 0 |
| 5 | Beta-CD-TDS | 17.3 | 60 | 15 |
| 6 | Beta-CD-TDS | 17.3 | 55 | 18 |

Examples 18-22

This group of examples shows that the angiogenesis suppression activity requires, aside from the characteristic complexing activity of the CD structure, a high water solubility. The CAM assays were made with a dosage of 50 µg to 60 µg of hydrocortisone in 10 µl of 0.45% methylcellulose in water.

In order to make comparisons that included the very water-soluble CD sulfates, the solubilities of which were so high at room temperature that measurement was not practical, all solubility measurements were made in liquid water at zero °C. One can picture this to be a measure of the competition of the hydrophile bonding with water in relation to the orderly bonding of water to itself. These comparisons are summarized in Table III.

Examples 18, 19 and 20 describe the results for the unsubstituted CDs, which gave no indication of angiogenesis suppression activity in the CAM assay. Example 21 shows the results for a sample of propoxylated β-CD (hydroxy-n-propyl ether) obtained from American Maize-Products Co. (Hammond, IN), reported to have an average of about 4 hydroxypropyl groups per CD molecule. Examples 22 shows the results obtained with the less sulfated β-CD product from Example 1(C) above. The results for Examples, 16, 17 and 5, including water solubilities at zero °C, are included to complete the comparison.

TABLE III

| CAM Assays | | | | | |
|---|---|---|---|---|---|
| Ex. No. | Compound | Conc. µg/10 µl | No. Examples | % Avascular zones | Solubility 0 °C, g/100 ml $H_2O$ |
| 18 | α-CD | 100<br>25 | 37<br>20 | 5± 0.3<br>0 | 6<br>6 |
| 19 | β-CD | 100<br>25 | 19<br>23 | 0<br>0.04 | 0.7<br>0.7 |
| 20 | γ-CD | 100 | | 0 | * |
| 21 | β-CD-propoxylated (~ 4 Pr) | 100<br>25 | 52<br>50 | 29±10.5<br>31± 9.7 | 20<br>20 |
| 21a | β-CD-14Me (~ 14 Meth) | 100<br>25 | 57<br>37 | 22± 5.7<br>20± 4.2 | 32 +<br>32 + |
| 22 | β-CD-(~ 7 $SO_4$) | 100<br>25 | 25<br>27 | 20± 9.6<br>8± 3.1 | 13<br>13 |
| 22a | β-CD (~ 4 propoxylated) (~ 14 $SO_4$) | 25 | | 37 | 39 |
| 16 | α-CD (~ 12 $SO_4$) | 100<br>25 | 40<br>25 | 17± 4.7<br>4± 2.7 | 36<br>36 |
| 17 | γ-CD (~ 16 $SO_4$) | 100<br>25 | 19<br>20 | 32± 5.3<br>19± 1.7 | 38<br>38 |
| 5 | β-CD-TDS | 100<br>50<br>25 | 101<br>75<br>107 | 55± 7.5<br>75± 5.8<br>58± 7.0 | 42<br>42<br>42 |
| * Insufficient material available for solubility determination. | | | | | |

Example 23

This example illustrates that Cortexolone with β-CD-TDS is an exceptionally effective antiangiogenic composition. Cortexolone is closely related to cortisone chemically. However, it possesses almost none of the functions of cortisone, except for the antiangiogenic effect in the presence of heparin.

Cortexolone, 50 µl/10 µl alone, gave zero % avascular zones in the CAM assay.

At the same dosage level, with 25 µg/10 µl of β-CD-TDS, the CAM assay showed 85% avascular zones of which 31% are (+ +) or greater.

Example 24

This example demonstrates that the angiostatic activity of L-2-azetidinecarboxylic acid, which has some inherent angiostatic activity, is strongly potentiated by the presence of CD-TDS. The results of the CAM assay, in the absence and presence of CD-TDS, are given in Table IV, Examples 24(a) and 24(b), respectively.

TABLE IV

| L-2-AZETIDINE | | | |
|---|---|---|---|
| | ug/10 µl | µg CD-TDS | % Avascular Zones |
| (a) L-2-Azetidine | 400 | 0 | 28 |
| (b) L-2-Azetidine | 400 | 25 | 100* |

* 50% of these avascular zones were 2+, and 25% were 3+, i.e., the largest avascular zones ever observed, greater than 10 mm.

Example 25

This example demonstrates the β-CD-TDS is about three times as effective as whole heparin in suppressing smooth muscle cell (SMC) growth when each is used alone (i.e., without exogenous corticosteroid or other supplementation). The bioassay of this activity was made using tissue cultures of rat aortic SMC and calf aortic SMC, with dosages ranging from 0.03 µg/ml up to 400 µg/ml.

The results are shown graphically in FIG. 2(A) and (B).

Example 26

This example demonstrates that topical administration of β-C-TDS in combination with hydrocortisone effectively inhibits neovascularization in the cornea.

The rabbit corneal test described by Perlin, Fed. Proc. 36:101 (1977) modified as described below, was employed to examine the effectiveness of a combination of β-CD-TDS and hydrocortisone In this corneal test, a sustained release polymer impregnated with bacterial endotoxin was first implanted in rabbit corneas. Endotoxin slowly released from this implant induces angiogenesis in the cornea in a manner analogous to the neovascularization observed in patients rejecting a corneal transplant. Rather than employing a second implant containing the test substance to deliver such test substance as generally used, in the present experiments the test substance was applied topically to the cornea, i.e., in the form of eye drops. Animals having received an endotoxin-containing implant were divided into four groups and were treated by topical application (eye drops) as follows:
Group 1, was not treated further and served as the control group; Group 2, received hydrocortisone alone (0.5 mg/ml); Group 3, hydrocortisone-21-phosphate and β-CD-TDS, (0.5 mg/ml and 1.0 mg/ml, respectively); and Group 4, β-CD-TDS (1.0 mg/ml). The diluent for the eye drops was saline in all cases. FIG. 3 (A-D) illustrates the results typically obtained on the 9th day post-implantation and treatment.

As shown in the photographs of FIG. 3, a combination of hydrocortisone and β-CD-TDS when topically applied to the cornea was very effective in inhibiting angiogenesis (FIG. 3C). The efficacy of this treatment is particularly apparent when the results obtained are compared to those observed in the untreated or control group (FIG. 3C vs FIG. 3A). In fact, in animals treated with hydrocortisone and β-CD-TDS, capillary growth was not only inhibited, but also new capillaries which had formed before initiation of the treatment regressed (FIG. 3C). On the other hand, hydrocortisone alone produced only a slight inhibition of angiogenesis (FIG. 3B). β-CD-TDS alone caused a slight stimulation of angiogenesis (FIG. 3D).

In another series of experiments, the antiangiogenic activity of a water-soluble cyclodextrin salt derivative in combination with cortexolone was evaluated using the rabbit cornea test as described by Gimbrone et al., J. Nat'l Cancer Inst. 52:413 (1974). In these experiments, E. coli endotoxin (17 µg/mm$^3$) incorporated into sustained release polymer pellets of ethylene vinylacetate copolymer [Elvax, Sigma Chemical, St. Louis, MO (hereinafter "Elvax")] was implanted between the vascular limbal edge of the rabbit corneas. The test substance(s) were administered by means of a second Elvax implant incorporating the particular test substance.

Elvax containing endotoxin was implanted into the corneas of 12 rabbits. The animals were then divided into 4 groups and 4 eyes were treated for each group as follows: Group 1, received no further treatment and served as the control group; Group 2, β-CD-TDS at 15 µg/mm$^3$ Elvax pellet; Group 3, cortexolone at 30 µg/mm$^3$ Elvax pellet; and Group 4, a combination of β-CD-TDS and cortexolone incorporated into an Elvax pellat at a final concentration of 15 µg/mm$^3$ Elvax of β-CD-TDS and 30 µg/mm$^3$ Elvax of cortexolone. The vessel length of new capillary growth was measured every 2 days with a slit-lamp stereoscope at 10X using an ocular grid calibrated to ± 0.1 mm.

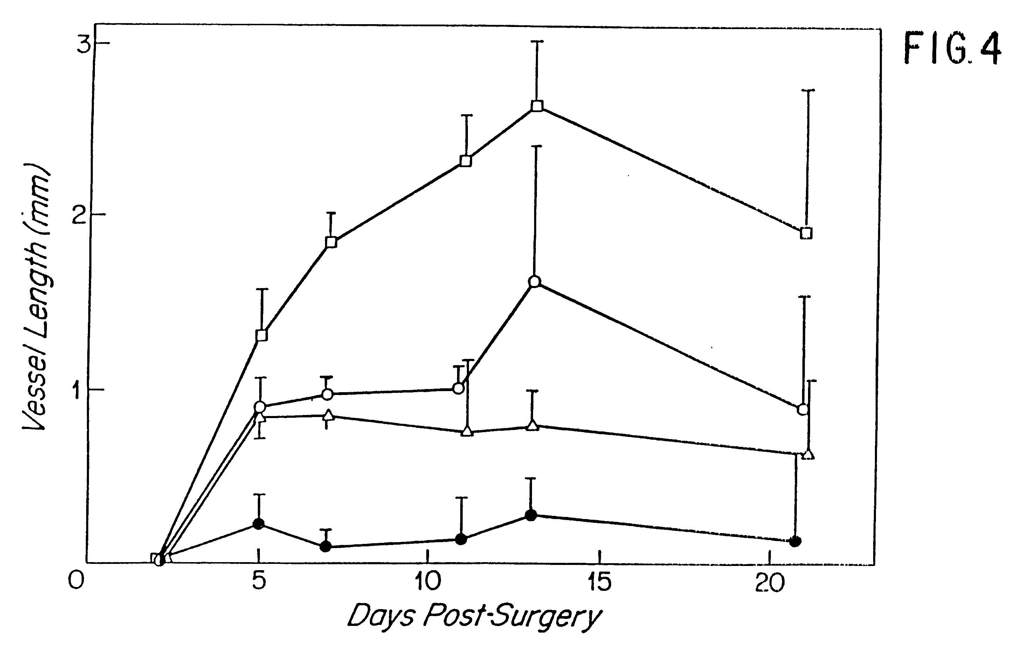

Measurement of vessel length alone underestimates the extent of antiangiogenic activity because such measurement does not assess capillary density. Thus, the vessel density was also evaluated and graded using the following scale: O = no vessels/cornea; 1 = 1-4 vessels/cornea; 2 = 5-20 vessels/cornea; 3 = 20-50 vessels/cornea; and 4 = more than 50 vessels/cornea. This grade was then multiplied by mean maximal length of the vessels to obtain a semi-quantitative estimate of vessel density (length-density index) for each cornea. Results obtained are graphically illustrated in FIG. 4 and tabulated in Table V.

As demonstrated in FIG. 4, the largest difference between treated and control corneas was observed on day 13 after implantation of the Elvax pellets. The mean vessel lengths and vessel density observed on day 13 are listed in Table V.

TABLE V

| INHIBITION OF ANGIOGENESIS IN CORNEAS | | | |
|---|---|---|---|
| | Inhibition (% of Untreated Control Group | | |
| | β-CD-TDS Cortexolone | Cortexolone Alone | β-CD-TDS Alone[a] |
| Vessel Length | 18% | 49% | 164% |
| Vessel Density | 8% | 61% | 303% |

[a] Percentage greater than 100% represents stimulation of vessel development.

As shown in Table V, a combination of β-CD-TDS and cortexolone inhibited linear capillary blood vessel growth to about 18% of that observed in untreated eyes. When capillary density was estimated, this combination suppressed vessel density to about 8% of that observed in untreated eyes. In contrast, as shown in Table V, when administered alone, cortexolone inhibited linear vessel growth only to about 49% and vessel density to about 61% that observed in untreated eyes. Surprisingly, as further demonstrated in Table V, administration of β-CD-TDS alone, stimulated vessel growth by about 164% and vessel density by about 303% above that observed in untreated eyes.

Based on these results, it is clear that administration of a cyclodextran salt derivative in combination with a steroid according to the present invention, is an effective method for inhibiting angiogenesis in ophthalmological tissues.

**Claims**

**1.** A composition for inhibiting undesired or pathological cell or tissue growth, including angiogenesis, in mammals, including humans, comprising (1) a derivative of α-,β- or γ-cyclodextrin in combination with (2) a latent growth-inhibiting steroid or a non-steroidal growth-inhibiting organic compound having inherent antiangiogenic activity, in which the derivative is an anionic salt derivative of said cyclodextrin having substituents selected from sulfate, phosphate, carboxylate and mixtures thereof associated with a physiologically acceptable cation, and has a solubility at 0 °C in distilled water at least 20 g/100 ml of water.

**2.** The composition according to claim 1, in which the derivative of α-,β-, or γ-cyclodextrin is a cyclodextrin sulfate.

**3.** The composition according to claim 2, in which the cyclodextrin sulfate is β -cyclodextrin tetradecasulfate.

**4.** The composition according to one of claims 1 to 3, in which the steroid has 17-alpha and 21-hydroxyl groups, 3- and 20-one groups and in the 16-position hydrogen, hydroxy or a methyl group and non-toxic, physiologically acceptable carboxylates, acetal, ketals and phosphates thereof.

**5.** The composition according to claim 4, in which the steroid is cortisone, hydrocortisone or cortexolone.

**6.** The composition according to claim 1, in which the non-steroidal growth-inhibiting organic compound is L-2-azetidinecarboxylic acid and the derivative of cyclodextrin is a cyclodextrin sulfate.

7. Use of a composition consisting of (1) a derivative or α-,β- or γ-cyclodextrin in combination with (2) a latent growth-inhibiting steroid or a non-steroidal growth-inhibiting organic compound having inherent antiangiogenic activity, the derivative being a salt consisting of an anionic derivative of a cyclodextrin having substituents selected from sulfate, phosphate, carboxylate and mixtures thereof associated with a non-toxic physiologically acceptable cation, characterized by a solubility at 0 °C in distilled water of at least 20 g/100 ml of water, for the preparation of a medicament for inhibiting undesired or pathological cell or tissue growth, including angiogenesis, in mammals, including humans.

8. Use of a composition consisting of (1) a derivative of α-,β- or γ-cyclodextrin in combination with (2) a latent growthinhibiting steroid or a non-steroidal growth-inhibiting organic compound having inherent antiangiogenic activity, the derivative being a salt consisting of an anionic derivative of a cyclodextrin having substituents selected from sulfate, phosphate, carboxylate and mixtures thereof associated with a non-toxic physiologically acceptable cation, characterized by a solubility at 0 °C in distilled water of at least 20 g/100 ml of water, for the preparation of a medicament for inhibiting angiogenesis in mammals, including humans.

9. The use according to claim 7, in which the salt of α-,β- or γ-cyclodextrin is a cyclodextrin sulfate.

10. The use according to claim 9, in which the salt is β-cyclodextrin sulfate.

11. The use according to claim 7, in which the steroid has 17-alpha and 21-hydroxyl groups, 3- and 20-one groups and in the 16-position hydrogen, hydroxy or a methyl group and non-toxic, physiologically acceptable carboxylates, acetal, ketals and phosphates thereof.

12. The use according to claim 7, in which the steroid is cortisone, hydrocortisone or cortexolone.

13. The use according to claim 7, in which the non steroidal growth-inhibiting organic compound is L-2-azetidinecarboxylic acid.

14. Products containing (1) a derivative of α-,β- or γ-cyclodextrin and (2) a latent growth-inhibiting organic compound having inherent antigiogenic activity as a combined preparation for simultaneous, separate or sequential use for inhibiting the pathological growth of smooth muscle cells in mammals, including humans, the derivative being a salt consisting of an anionic derivative of a cyclodextrin having substituents selected from the group consisting of sulfate, phosphate, carboxylate and mixtures thereof associated with a non-toxic physiologically acceptable cation, characterized by a solubility of at least 20 g/100 ml of water.

**Patentansprüche**

1. Zusammensetzung zur Inhibierung von unerwünschtem oder pathologischem Zell- oder Gewebewachstum, einschließlich Angiogenese, in Säugetieren, einschließlich Menschen, umfassend (1) ein Derivat von α-, β- oder γ-Cyclodextrin in Kombination mit (2) einem latenten wachstumsinhibierenden Steroid oder einer nicht-steroiden wachstumsinhibierenden organischen Verbindung mit inhärenter antiangiogener Wirkung, wobei das Derivat ein Salz aus einem anionischen Cyclodextrinderivat mit Substituenten ausgewählt aus Sulfat, Phosphat, Carboxylat und Mischungen davon in Assoziation mit einem nicht-toxischen physiologisch annehmbaren Kation ist, und bei 0 °C in destilliertem Wasser eine Löslichkeit von mindestens 20 g/100 ml Wasser besitzt.

2. Zusammensetzung nach Anspruch 1, dadurch gekennzeichnet, daß das Derivat von α-, β-, oder γ-Cyclodextrin ein Cyclodextrinsulfat ist.

3. Zusammensetzung nach Anspruch 2, dadurch gekennzeichnet, daß das Cyclodextrinsulfat β-Cyclodextrintetradecasulfat ist.

4. Zusammensetzung nach einem der Ansprüche 1 bis 3, dadurch gekennzeichnet, daß das Steroid 17-alpha- und 21-Hydroxyl-Gruppen, 3- und 20-On-Gruppen und in 16-stellung Wasserstoff, Hydroxy oder eine Methylgruppe aufweist, und nicht-toxische, physiologisch annehmbare Carboxylate, Acetale, Ketale und Phosphate davon.

5. Zusammensetzung nach Anspruch 4, dadurch gekennzeichnet, daß das Steroid Cortison, Hydrocortison oder Cortexolon ist.

6. Zusammensetzung nach Anspruch 1, dadurch gekennzeichnet, daß die nicht-steroide wachstumshemmende organische Verbindung L-2-Azetidincarbonsäure und das Cyclodextrinderivat Cyclodextrinsulfat ist.

7. Verwendung einer Zusammensetzung aus (1) einem Derivat von α-, β- oder γ-Cyclodextrin in Kombination mit (2) einem latenten wachstumshemmenden Steroid oder einer nichtsteroiden wachstumshemmenden organischen Verbindung mit inherenter antiangiogener Wirkung, wobei das Derivat ein Salz aus einem anionischen Cyclodextrinderivat mit substituenten ausgewählt aus Sulfat, Phosphat, Carboxylat und Mischungen davon in Assoziation mit einem nicht-toxischen physiologisch annehmbaren Kation ist, und durch eine Löslichkeit bei 0°C in destilliertem Wasser von mindestens 20 g/100 ml Wasser gekennzeichnet ist, zur Herstellung eines Arzneimittels zur Hemmung von unerwünschtem oder pathologischem Zell- oder Gewebewachstum, einschließlich Angiogenese, in Säugetieren, einschließlich Menschen.

8. Verwendung einer Zusammensetzung aus (1) einem Derivat von α-, β-, oder γ-Cyclodextrin in Kombination mit (2) einem latenten wachstumshemmenden Steroid oder einer nichtsteroiden wachstumshemmenden organischen Verbindung mit inhärenter antiangiogener Wirkung, wobei das Derivat ein Salz bestehend aus einem anionischen Cyclodextrinderivat mit Substituenten ausgewählt aus Sulfat, Phosphat, Carboxylat und Mischungen davon assoziiert mit einem nicht-toxischen physiologisch annehmbaren Kation ist, und durch eine Löslichkeit bei 0°C in destilliertem Wasser von mindestens 20 g/100 ml Wasser gekennzeichnet ist, zur Herstellung eines Arzneimittels zur Hemmung von Angiogenese in Säugetieren, einschließlich Menschen.

9. Verwendung nach Anspruch 7, dadurch gekennzeichnet, daß das Salz von α-, β- oder γ-Cyclodextrin ein Cyclodextrinsulfat ist.

10. Verwendung nach Anspruch 9, dadurch gekennzeichnet, daß das Salz β-Cyclodextrinsulfat ist.

11. Verwendung nach Anspruch 7, dadurch gekennzeichnet, daß das Steroid 17-alpha- und 21-Hydroxyl-Gruppen, 3- und 20-On-Gruppen und in 16-Stellung Wasserstoff, Hydroxy oder eine Methylgruppe aufweist, und nicht-toxische, physiologisch annehmbare Carboxylate, Acetale, Ketale und Phosphate davon.

12. Verwendung nach Anspruch 7, dadurch gekennzeichnet, daß das Steroid Cortison, Hydrocortison oder Cortexolon ist.

13. Verwendung nach Anspruch 7, dadurch gekennzeichnet, daß die nicht-steroide wachstumshemmende organische Verbindung L-2-Azetidincarbonsäure ist.

14. Produkt enthaltend (1) ein Derivat von α-, β-oder γ-Cyclodextrin und (2) eine latente wachstumshemmende organische Verbindung mit inhärenter antiangiogener Wirkung als Kombinationspräparat zur gleichzeitigen, getrennten oder hintereinanderfolgenden Verwendung zur Hemmung des pathologischen Wachstums von Zellen der glatten Muskulatur in Säugetieren, einschließlich Menschen, wobei das Derivat ein Salz ist, daß aus einem anionischen Cyclodextrinderivat mit Substituenten ausgewählt aus der Gruppe bestehend aus Sulfat, Phosphat, Carboxylat und Mischungen davon assoziiert mit einem nicht-toxischen physiologisch annehmbaren Kation ist, und durch eine Löslichkeit von mindestens 20 g/100 ml Wasser gekennzeichnet ist.

**Revendications**

1. Composition pour inhiber une croissance indésirable ou pathologique de cellules ou de tissus, y compris l'angiogenèse, chez des mammifères, y compris l'homme, comprenant (1) un dérivé d'α-, β-ou γ-cyclodextrine en combinaison avec (2) un stéroïde inhibiteur latent de croissance ou un composé organique inhibiteur de croissance non stéroïdien ayant une activité antiangionénique inhérente, dans laquelle ledit dérivé est un dérivé de type sel anionique de ladite cyclodextrine, ayant des substituants

choisis parmi sulfate, phosphate, carboxylate et leurs mélanges, associé à un cation physiologiquement acceptable, et ayant une solubilité dans l'eau distillèe à 0 ° C d'au moins 20 g/100 ml d'eau.

2. Composition selon la revendication 1, dans laquelle le dérivé d'α-, β- ou γ-cyclodextrine est un sulfate de cyclodextrine.

3. Composition selon la revendication 2, dans laquelle le sulfate de cyclodextrine est le tétradécasulfate de β-cyclodextrine.

4. Composition selon l'une quelconque des revendications 1 à 3, dans laquelle le stéroïde a des groupes 17-α- et 21-hydroxy, des groupes 3- et 20-one et, dans la position 16, un atome d'hydrogène ou un groupe hydroxy ou méthyle, et les carboxylates, acétals, cétals et phosphates de celui-ci non toxiques, physiologiquement acceptables.

5. Composition selon la revendication 4, dans laquelle le stéroïde est la cortisone, l'hydrocortisone ou la cortexolone.

6. Composition selon la revendication 1, dans laquelle le composé organique inhibiteur de croissance non stéroïdien est l'acide L-2-azétidinecarboxylique et le dérivé de cyclodextrine est un sulfate de cyclodextrine.

7. Utilisation d'une composition constituée de (1) un dérivé d'α-, β- ou γ-cyclodextrine en combinaison avec (2) un stéroïde inhibiteur latent de croissance ou un composé organique inhibiteur de croissance non stéroïdien ayant une activité antiangiogénique inhérente, dans laquelle ledit dérivé est un sol constitué d'un dérivé anionique de ladite cyclodextrine, ayant des substituants choisis parmi sulfate, phosphate, carboxylate et leurs mélanges, associé à un cation non toxique, physiologiquement acceptable, caractérisé par une solubilité dans l'eau distillée à 0 ° C d'au moins 20 g/100 ml d'eau, pour la préparation d'un médicament pour inhiber une croissance indésirable ou pathologique de cellules ou de tissus, y compris l'angiogenèse, chez des mammifères, y compris l'homme.

8. Utilisation d'une composition constituée de (1) un dérivé d'α-, β- ou γ-cyclodextrine en combinaison avec (2) un stéroïde inhibiteur latent de croissance ou un composé organique inhibiteur de croissance non stéroïdien ayant une activité antiangionénique inhérente, dans laquelle ledit dérivé est un sel constitué d'un dérivé anionique de ladite cyclodextrine, ayant des substituants choisis parmi sulfate, phosphate, carboxylate et leurs mélanges, associé à un cation non toxique, physiologiquement acceptable, caractérisé par une solubilité dans l'eau distillée à 0 ° C d'au moins 20 g/100 ml d'eau, pour la préparation d'un médicament pour inhiber l'angiogenèse chez des mammifères, y compris l'homme.

9. Utilisation selon la revendication 7, dans laquelle le sel d'α-, β- ou γ-cyclodextrine est un sulfate de cyclodextrine.

10. Utilisation selon la revendication 9, dans laquelle le sel est le sulfate de β-cyclodextrine.

11. Utilisation selon la revendication 7, dans laquelle le stéroïde a des groupes 17-α- et 21-hydroxy, des groupes 3- et 20-one et, dans la position 16, un atome d'hydrogène ou un groupe hydroxy ou méthyle, et les carboxylates, acétals, cétals et phosphates de celui-ci non toxiques, physiologiquement acceptables.

12. Utilisation selon la revendication 7, dans laquelle le stéroïde est la cortisone, l'hydrocortisone ou la cortexolone.

13. Utilisation selon la revendication 7, dans laquelle le composé organique inhibiteur de croissance non stéroïdien est l'acide L-2-azétidinecarboxylique.

14. Produits contenant (1) un dérivé d'α-, β- ou γ-cyclodextrine et (2) un composé organique inhibiteur latent de croissance ayant une activité antiangiogénique inhérente sous forme d'une préparation combinée pour l'utilisation simultanée, séparée ou séquentielle pour inhiber la croissance pathologique des cellules musculaires lisses chez les mammifères, y compris l'homme, le derivé étant un sel

constitué d'un dérivé anionique d'une cyclodextrine, ayant des substituants choisis dans le groupe constitué par sulfate, phosphate, carboxylate et leurs mélanges, associé à un cation non toxique physiologiquement acceptable, caractérisé par une solubilité d'au moins 20 g/100 ml d'eau.

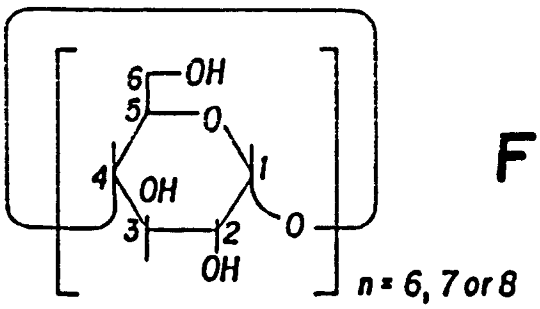

FIG. 1A

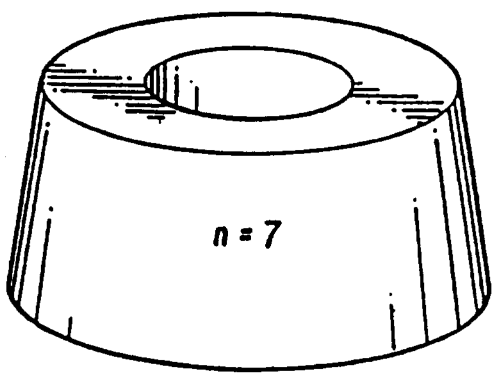


FIG. 1B

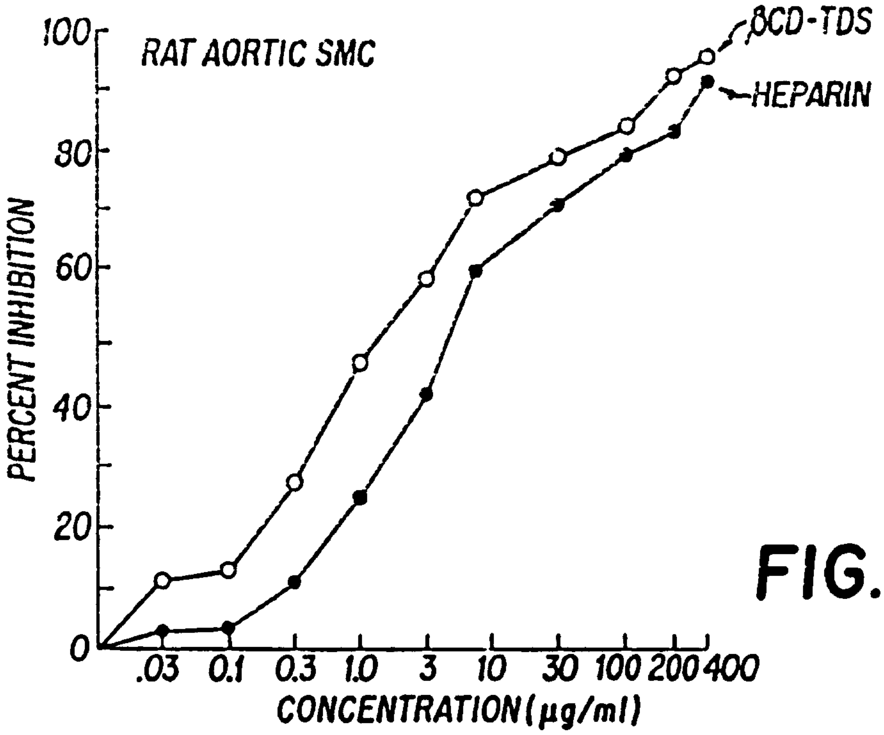
100
80
60
40
20
0
PERCENT INHIBITION
RAT AORTIC SMC
βCD-TDS
HEPARIN
.03 0.1 0.3 1.0 3 10 30 100 200 400
CONCENTRATION (μg/ml)

FIG. 2A

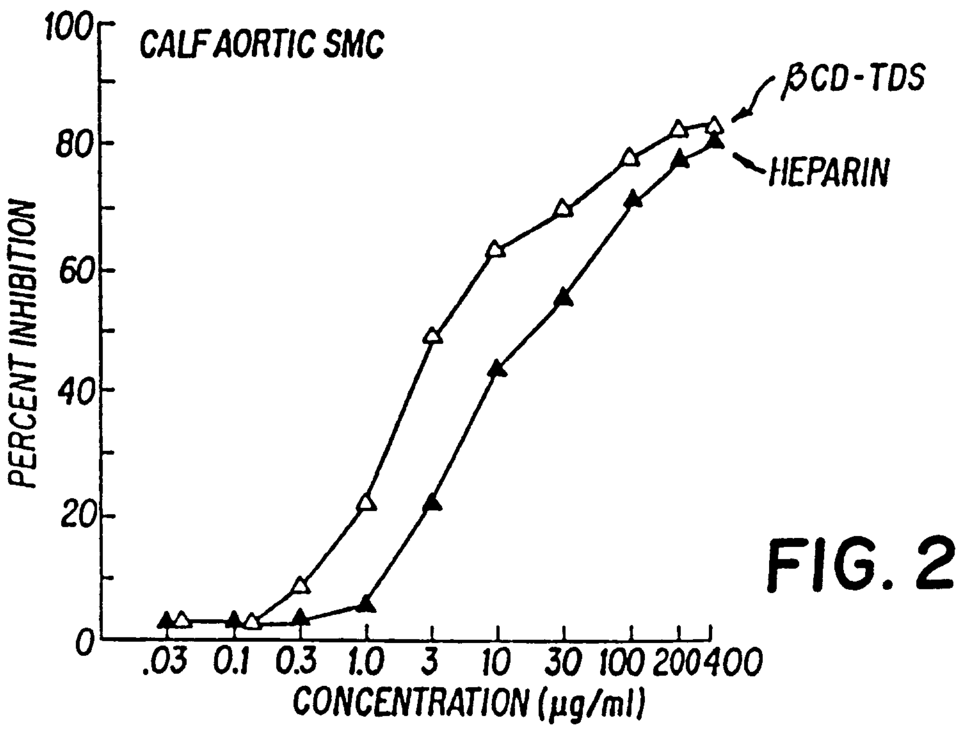
100
80
60
40
20
0
PERCENT INHIBITION
CALF AORTIC SMC
βCD-TDS
HEPARIN
.03 0.1 0.3 1.0 3 10 30 100 200 400
CONCENTRATION (μg/ml)

FIG. 2B

FIG. 3A

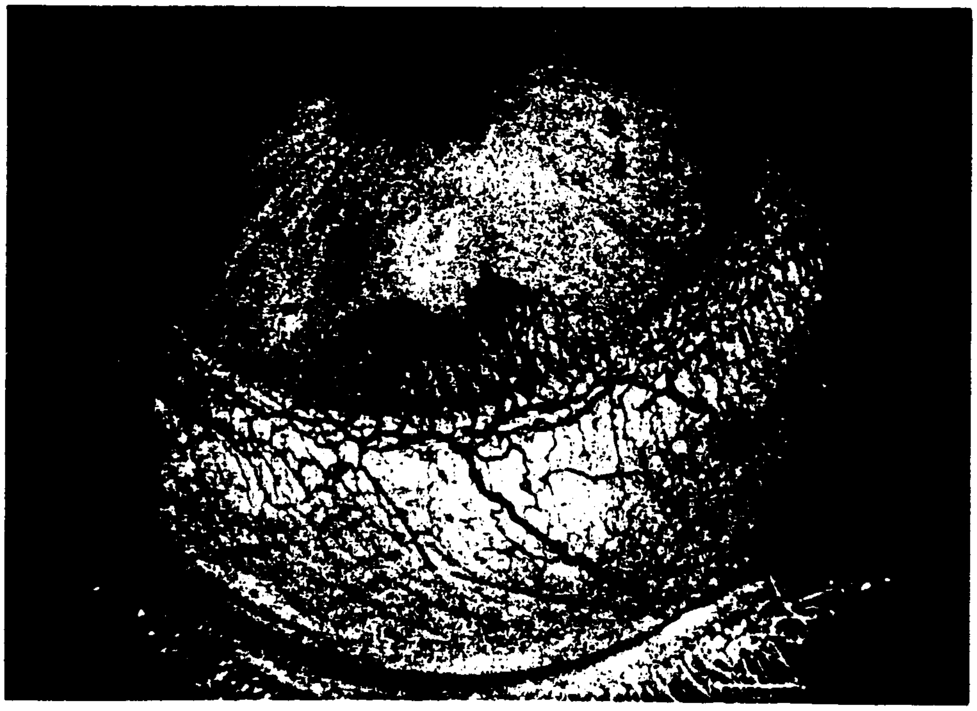

FIG. 3B

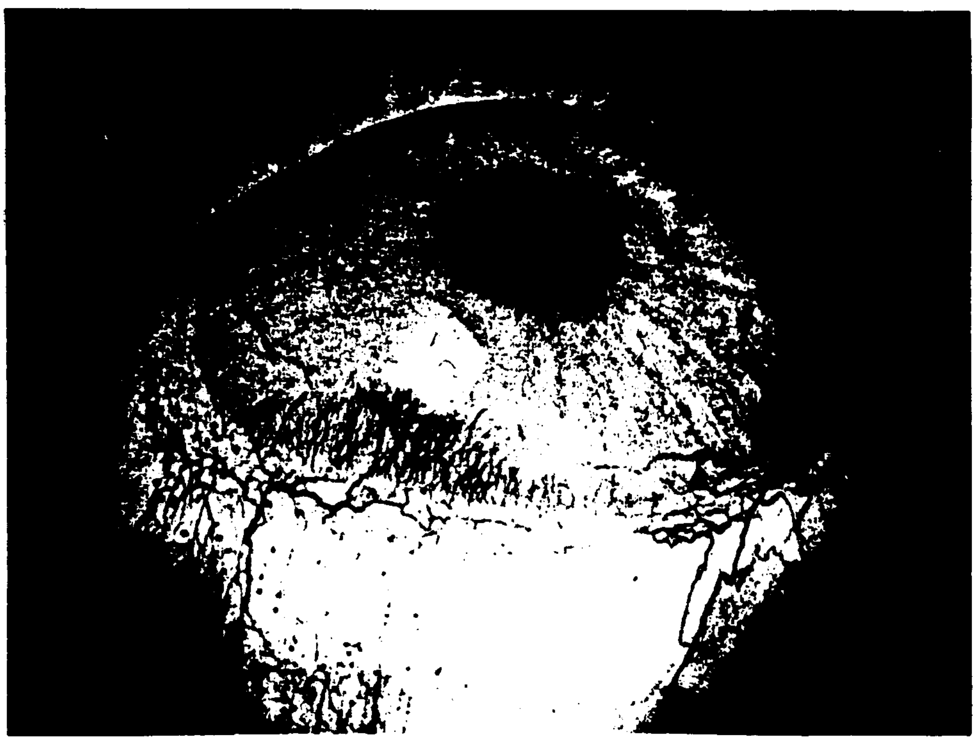

FIG. 3C

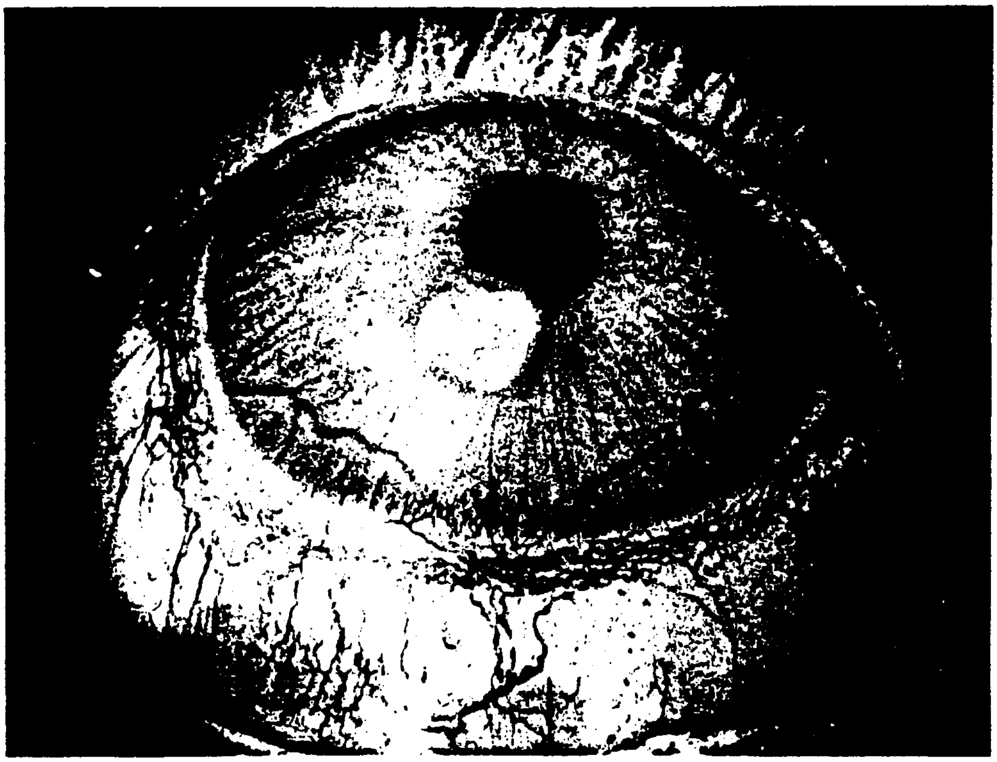

FIG. 3D

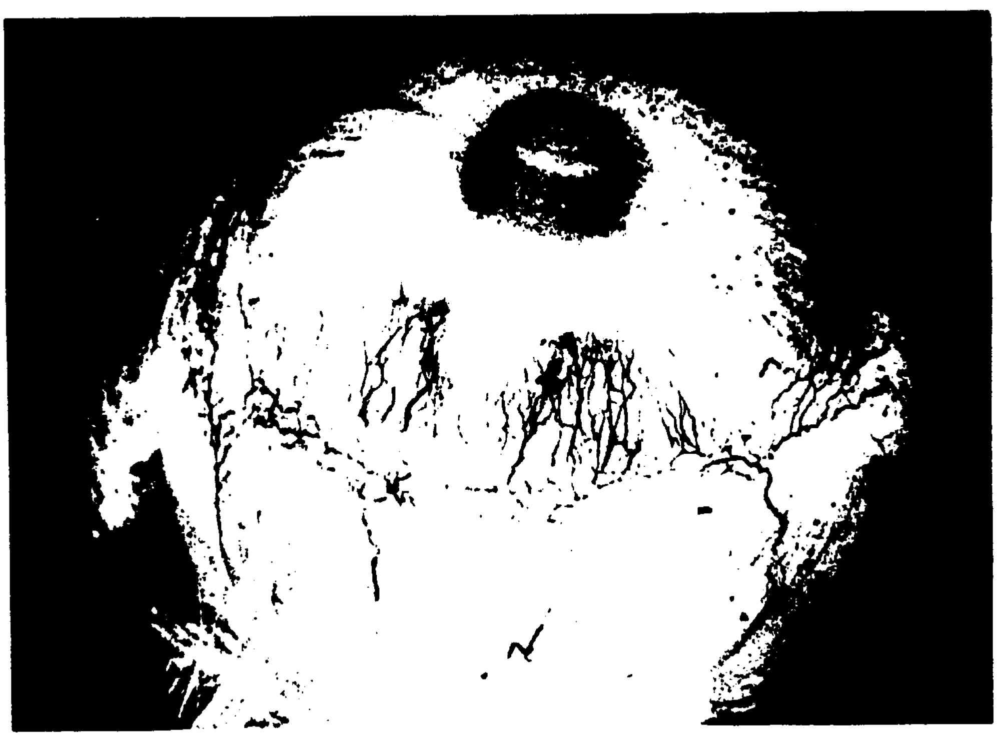

FIG. 4